(19) Europäisches Patentamt
European Patent Office
Office européen des brevets

(11) **EP 1 267 833 B1**

(12) **EUROPEAN PATENT SPECIFICATION**

(45) Date of publication and mention
of the grant of the patent:
**16.06.2004 Bulletin 2004/25**

(51) Int Cl.⁷: $A61K\ 9/113$, $A61K\ 9/16$, $A61K\ 9/51$

(21) Application number: **01917326.9**

(22) Date of filing: **05.04.2001**

(86) International application number:
**PCT/GB2001/001562**

(87) International publication number:
**WO 2001/074332 (11.10.2001 Gazette 2001/41)**

(54) **PHARMACEUTICAL PREPARATIONS AND THEIR MANUFACTURE**

ARZNEIZUBEREITUNGEN UND DEREN HERSTELLUNGSVERFAHREN

PREPARATIONS PHARMACEUTIQUES ET LEUR FABRICATION

(84) Designated Contracting States:
**AT BE CH CY DE DK ES FI FR GB GR IE IT LI LU MC NL PT SE TR**

(30) Priority: **05.04.2000 GB 0008411**

(43) Date of publication of application:
**02.01.2003 Bulletin 2003/01**

(73) Proprietor: **Vectura Limited**
**Chippenham, Wiltshire SN14 6FH (GB)**

(72) Inventors:
• **STANIFORTH, John Nicholas**
  **Bath BA2 2AT (GB)**
• **TOBYN, Michael John**
  **Wiltshire BA14 9SS (GB)**
• **CLINCH, Cheryl Julia**
  **Bristol BS4 2LL (GB)**
• **PRICE, Robert**
  **Bath BA2 3RT (GB)**

(74) Representative: **Jump, Timothy John Simon et al**
**Venner Shipley & Co.**
**20 Little Britain**
**London EC1A 7DH (GB)**

(56) References cited:
**EP-A- 0 275 796**          **EP-A- 0 804 952**
**WO-A-90/15593**

**Description**

The invention relates to particles comprising a therapeutic substance and to methods of making such particles.

It is common in the manufacture of solid pharmaceutical products to precipitate one or more ingredients of the product as solid particles from solution by rapidly cooling the solution or by adding a precipitant material, such as a non-solvent. Whilst such processes are generally rapid they suffer from the disadvantage that it is difficult to control reproducibly the characteristics, such as the size, shape and crystal form of the solid particles formed. Those characteristics can influence not only the handling properties of the particles but also the speed of dissolution and delivery of the pharmaceutical substance in the body. Furthermore, some applications require particles of specified characteristics, for example, pharmaceutical particles for use in dry powder inhalers should be less than 10μm and preferably less than 5μm in diameter. It is also known that the size, shape and crystal form of pharmaceutical particles can influence the compressibility of those particles which in turn is an important factor in determining the conditions required successfully to produce tablets comprising the particles. It is therefore desirable to be able to control the crystallisation process to provide particles which have the required characteristics. In particular, it is desirable to obtain particles having a relatively narrow distribution of particle size, for example, because such particles will have more clearly defined and predictable dissolution characteristics as compared to particles of the same material having a relatively broad particle size distribution. Whilst it is possible to produce relatively fine cuts (i.e. narrow distributions) of large particles by, for example, sieving, that is wasteful of particles not falling into the desired cut and furthermore sieving is not suitable for particles smaller than 30μm.

In a method known as the emulsion solvent diffusion method, a solution of a drug in a first solvent is poured into a second solvent in which the drug is insoluble with stirring to form droplets of the drug solution in the second solvent. The two solvents are miscible and so the first solvent diffuses into the second solvent and vice versa thereby causing the drug to precipitate. This process is a variation of the common technique of precipitation of a solute by pouring a solution into a non-solvent. No stable emulsion is formed and the formation of the particles occurs simultaneously with the diffusion of the first solvent from the droplets.

It is also known to spray dry an emulsion of an organic substance in water to provide the organic substance as a dry powder. In such processes the formation of the particles occurs during the loss of the water and any solvent present with the consequent destruction of the emulsion and it is believed that that makes such crystallisation difficult to control in a way which would enable reproducible preparation of particles having the desired characteristics.

WO90/15593 discloses a method in which an organic solution of a water insoluble drug is emulsified in an aqueous phase. The organic solvent is removed, for example by heating to cause evaporation thereby causing precipitation of the drug to form a suspension of drug particles in the aqueous phase. Again, the formation of particles occurs during the loss of the solvent from the droplets and the consequent destruction of the emulsion droplets.

There remains a need for a process of preparing particles comprising a therapeutic substance which allows more control over the formation of the particles and gives more reproducible results from batch-to-batch and from the small scale to the large scale.

There is also a need for particles comprising a therapeutically active substance which have improved properties such as crystallinity and particle size distribution.

The invention provides a method of making a composition comprising active particles comprising an active substance, the method comprising the steps of:

a) providing an emulsion having a dispersed phase comprising a solution of the active substance in a solvent; and
b) inducing the formation, in the emulsion, of solid particles comprising the active substance, thereby forming an emulsion comprising the solid active particles.

The method of the invention enables characteristics of the solid particles produced to be controlled in two ways. Firstly the nature of the emulsion can be adjusted as desired. Emulsions are used in technical fields such as emulsion polymerisation and the control of emulsion properties in those fields is known. For example, the mean droplet size can be influenced by such factors as the type and concentration of surfactant used, the rate of agitation and the composition of the dispersed and continuous phases. Typically, in the method of the present invention, an emulsion having a smaller mean droplet size will produce particles of a smaller size than an emulsion having a larger droplet size. The shape of the particles may be influenced by the degree of supersaturation within the droplets during nucleation and subsequent crystal growth. The ability reproducibly to form emulsions having desired characteristics leads, in turn, to greater reproducibility and consistency in the characteristics of the active particles produced as compared to the prior art processes involving rapid crystallisation or precipitation. Another way in which the nature of the emulsion can be adjusted is that the viscosity can be selected as desired, for example, the viscosity of the emulsion may be made lower than the viscosity of the solution of active substance, especially where the solution is concentrated and/or the active substance is of high molecular weight, for example, a polypeptide. Alternatively, an emulsion of higher viscosity may be used, for example, an emulsion which is stabilised by a polymeric thickener such as xanthan gum. Thus, it can be seen that it is possible, in the method of the invention, to adjust important variables such as the viscosity which influence the rate of mass and/or heat transfer and therefore influence the processes of crystallisation and precipitation.

Secondly, control of the rate at which the environment within the dispersed phase changes allows control of the rate of growth of the particles. As the dispersed phase of an emulsion has a very high surface area to volume ratio, processes which depend on mass or heat transfer across the phase boundary can be finely controlled and that allows a fine control of the rate at which the particles are induced to nucleate and grow.

It is a particular advantage of the method of the invention that improved reproducibility between batches is obtainable as compared with prior art methods.

The methods by which the active particles are induced to form do not rely on the removal of the solvent in which the active substance is dissolved from the emulsion. It is believed that removal of the solvent from the emulsion, for example, by evaporation, disturbs the emulsion in a way which makes it more difficult to obtain the desired high levels of crystallinity and low polydispersity of the particle thereby making it difficult to reliably reproduce particles having the desired characteristics. In the method of the invention, the solvent substantially remains within the emulsion, although it may diffuse, for example, from the internal disperse phase of a multiple three phase emulsion to the outer phase. Preferably, however, the solvent will substantially remain within the dispersed phase.

After formation of the particles the emulsion will be a complex mixture of the solvent, the continuous phase and the active particles. The active particles may be present in the dispersed phase, the continuous phase and/or at the interface between the two phases. The term "emulsion" will be understood to include compositions comprising a dispersed phase, a continuous phase and active particles.

Preferably, the method is a continuous process. Advantageously, the method is operated as a semi-continuous process. Those method are preferred since such continuous or semi-continuous processes are generally thought to give a more consistent product than batch processes.

The term "active substance" is used herein to mean a therapeutically active substance. The term "therapeutically active substance" should be taken to include substances having a prophylactic activity. The active substance may be, for example, an antibiotic, an analgesic, or an anaesthetic. The dispersed phase may comprise a mixed solution of more than one active substance provided that the active substances are soluble in the same solvent. In that case particles of mixed composition will be formed.

Where the active particles are intended for use in an inhaler, the active substance may be a substance for the treatment of respiratory disease, such as a $\beta_2$-agonist, for example, a compound selected from terbutaline, salbutamol, salmeterol and formoterol. If desired, the active particles may comprise more than one of those active substances. References herein to any active substance are to be understood to include any physiologically acceptable derivative. In the case of the $\beta_2$-agonists mentioned above, physiologically acceptable derivatives include especially salts, including sulphates. Preferably, the active particles are particles of salbutamol sulphate. The active substance may be an anti-muscarinic which may be ipatropium bromide or may be a salt of tiatropium or glycopyrrolate. The active substance may be a steroid, which may be beclomethasone dipropionate or may be fluticasone. The active substance may be a cromone which may be sodium cromoglycate or nedocromil. The active substance may be a leukotriene receptor antagonist. The active substance may be a carbohydrate, for example heparin. The active substance for inhalation may advantageously be a pharmacologically active substance for systemic use provided that it is capable of being absorbed into the circulatory system via the lungs.

The active particles may comprise peptides or polypeptides or proteins such as DNase, leukotrienes or insulin (including substituted insulins and pro-insulins), cyclosporin, interleukins, cytokines, anti-cytokines and cytokine receptors, vaccines (including influenza, measles, 'anti-narcotic' antibodies, meningitis), growth hormone, leuprolide and related analogues, interferons, desmopressin, immunoglobulins, erythropoeitin and calcitonin. Insulin is a preferred active substance.

The active substance may be suitable for oral administration. The active substance for oral administration may be one of the active substances for systemic use as mentioned above. The active substance may be a substance which is of low solubility under conditions obtaining in parts of the digestive tract, for example calcium carbonate, bismuth subnitrate and magnesium trisilicate.

Organic compounds which may be used as an active substance in accordance with the invention include, for example all products of combinatorial chemistry, rosiglitazone and other glitazone drugs, hydrochlorothiazide, griseofulvin, lamivudine and other nucleoside reverse transciptase inhibitors, simvastatin and other statin drugs, bezafibrate and other fibrate drugs and loratidine, and any physiologically tolerable salts or derivatives thereof.

Usually the emulsions used in the method of the invention comprise small droplets (the dispersed phase) of the solution of the active substance dispersed in a continuous liquid phase. The emulsions may be multiple emulsions, for example, a water-in-oil-in-water emulsion. Multiple emulsions may comprise two or more dispersed phases. In a multiple emulsion the solution of the active substance may be for example, an aqueous solution, dispersed in an oil phase which is itself dispersed in an aqueous phase. Alternatively, the active substance may be in the oil phase. Such multiple emulsions are described by Florence, A.T. and Whitehill, D., (The Formulation and Stability of Multiple Emulsions, Int. J. Pharmaceut. 11, 227-308 (1982)). It will be understood that the term 'continuous phase' as used herein will, when the emulsion is a multiple emulsion, be taken to refer to the phase which surrounds the dispersed phase comprising the solution of the active substance, even though in a multi-phase emulsion that continuous phase may itself be dispersed. Bicontinuous emulsions, in which both phases are continuous, may be suitable for use in the method of the invention. Where a bicontinuous emulsion is used, it will be understood that the term dispersed phase refers to the phase comprising the active substance.

To provide the emulsion the active substance will usually be combined with the solvent to form the solution, which will then be combined with the continuous phase to form the emulsion. If a three phase emulsion is desired such as a water-in-oil-in-water emulsion, the third phase may then be added to the emulsion to form the three phase emulsion.

Preferably, the emulsions are stable before and during the formation of the active particles, that is, the dimensions of the droplets of the dispersed phase are substantially constant and/or the emulsions show no substantial phase separation over a period of at least 5 minutes, preferably one hour. This enables, where desired, crystallisation to take place over a relatively long time period thereby giving improved control of the process.

The dispersed phase may be in the form of droplets having a mean droplet diameter of at least 1μm. Preferably the droplets have a mean diameter of 1 to 100μm, advantageously 1 to 20μm. Droplet diameters may be measured by microscopy. Where a mean value is desired, the diameter is preferably measured by light scattering, using for example, a Malvern Mastersizer. Alternatively, the emulsion may be.a microemulsion, that is, the dispersed phase is in the form of droplets typically having a diameter of less than 1μm. Such emulsions are particularly suitable for the preparation of small active particles, for example, active particles having a diameter of less than 1μm. Unless indicated otherwise, the word diameter as used herein should be taken to mean mass median diameter.

Advantageously, the emulsion comprises one or more surfactants. The surfactants may be anionic, cationic or nonionic and will advantageously by physiologically acceptable. Suitable nonionic surfactants include sorbitan fatty acid esters, polyoxyethylene sorbitan fatty acid esters, poloxamers, cetostearyl alcohol, glycerol palmitostearate, lecithin, polyoxyethylene caster oil derivatives, polyoxyethylene stearates and alkyl ether ethoxylates. Suitable anionic surfactants include sodium lauryl sulphate, sodium dioctyl sulphosuccinate, ether carboxylates. Dodecyl trimethyl ammonium bromide is a suitable cationic surfactant.

Where the emulsion is a microemulsion, the surfactant is preferably a dual hydrocarbon chain surfactant such as didodecyldimethylammonium bromide (DDAB) or sodium dioctyl sulphosuccinate. DDAB is, however, less preferred as it is not physiologically acceptable and residual DDAB must therefore be carefully removed from the active particles, for example, by washing.

In emulsions comprising little or no surfactant, the interfacial free energy (of the boundary surface between the phases) is high which is believed to have the effect of discouraging nucleation of crystals at that interface. In such an emulsion, therefore, it is to be expected that nucleation will occur in the interior of the droplet, with the solid particle growing outwards towards the interface. Alternatively, in emulsions where the surfactant is present in an amount effective to lower the interfacial free energy, it is expected that nucleation may occur at the interface, with the solid particle growing inwards. Thus, it is to be expected that different particle morphologies may be obtained by varying the quantity and/or type of surfactant present.

As noted above, nucleation and growth of the active particles may occur at the interface between the dispersed and continuous phases or in the interior of the dispersed phase. Once the active particles have been formed, they may remain in the disperse phase or at the interface or they may migrate into the continuous phase. Following the formation of the active particles the emulsion will usually comprise the active particles, the continuous phase and the solvent.

Usually, the solvent is a liquid which is substantially insoluble in the continuous phase. Advantageously, the solvent and the liquid making up the continuous phase are immiscible.

Preferably, the dispersed phase is an aqueous phase (a water-in-oil emulsion), that is, the solvent for the active substance is water. This has the advantage that water is physiologically acceptable and therefore traces of water remaining in the active particles are not a cause for concern and in any case can normally easily be removed by drying. Alternatively, especially if the active substance is not soluble in water, the solvent may be an organic solvent such as a low viscosity water immiscible organic solvent such as n-hexane or the organic solvent may be an oil, for example, a vegetable oil or a mineral oil. The solvent may be a mixture of more than one such organic solvent. Where the solvent is an organic solvent, the continuous phase may be an aqueous phase. Suitable solvents include soybean oil, sesame oil, olive oil, safflower oil, cotton seed oil, corn oil, hydrogenated vegetable oil, hydrogenated caster oil, mineral oil, paraffin, cyclohexane, toluene, octadecanol, tetradecane, hexadecane, carbon tetrachloride. Preferably, the organic solvent is physiologically acceptable. If the organic solvent is not physiologically acceptable then any residual solvent remaining on isolated active particles should be removed, for example, by washing with a liquid which is miscible with the solvent but is a non-solvent for the active substance.

The solvent will usually not crystallise or precipitate with the active material and will therefore not form any substantial part of the active particles although some residual solvent may be present in those active particles.

The concentration of the active substance in the solution will be limited by the solubility of the active substance in the solvent but may range from 0.1% to 90% w/w, preferably from 0.2 to 60%, more preferably from 1 to 50%, and it may especially advantageously be from 1 to 30%.

Advantageously, the step of inducing the active substance to form solid particles involves changing the temperature of the emulsion. That method is suitable where the solubility of the active substance in the solvent increases with temperature thereby allowing the emulsion to be prepared using a concentrated solution of the active substance at an elevated temperature. Reducing the temperature of the emulsion will induce crystallisation of the active substance to form particles. Advantageously, the rate of change of temperature is controlled.

Preferably, the step of inducing the formation, in the dispersed phase, of solid particles involves the addition of a precipitant substance. The precipitant substance may be any substance that, when added to the emulsion, causes the active substance to precipitate or crystallise out of solution. Advantageously, the composition of the precipitant substance is chosen and/or the rate of addition of the precipitant substance is controlled so as to produce solid particles having the desired characteristics.

Preferably, the precipitant substance is a substance which will combine with the active substance to form an insoluble product comprising the active substance. Such a substance may be, for example, an ionic substance which combines with the active substance to form an insoluble salt.

Preferably, the addition of the precipitant substance causes a change in the pH of the emulsion. The precipitant substance may be an acid or a base. For example, where the active substance is in the form of a water soluble carboxylic acid salt and the solvent is water, the addition of a stronger acid will induce the precipitation of the insoluble acid form of the active substance.

It will be understood that, where the addition of the precipitant substance induces the precipitation of an insoluble derivative of the active substance, the term active substance as used herein includes both the soluble form and the insoluble derivative.

Preferably the precipitant substance is a liquid which is miscible with the solvent but which is a non-solvent for the active substance. As the non-solvent precipitant substance is added to the emulsion, it diffuses into the dispersed phase rendering the active substance less soluble in the dispersed phase. For example, where the active substance is a polypeptide or protein, the precipitant substance may be a liquid of high ionic strength, such as a solution of a salt.

Advantageously the step of inducing the formation, in the dispersed phase, of solid particles involves the addition of a second emulsion, the dispersed phase of the second emulsion comprising the precipitant material. The composition of the continuous phases of the emulsions is preferably the same so that the emulsions are easily mixed and problems of incompatibility are avoided. In this way a precipitant substance which is not soluble in the continuous phase of the emulsion can be utilised. The mixed emulsion will then comprise a mixture of droplets comprising the active substance and droplets comprising the precipitant substance. Exchange of solutes between the droplets through the continuous phase will lead to the formation of solid particles comprising the active substance as described below.

Preferably, the step of inducing the formation, in the dispersed phase, of solid particles involves the use of ultrasonic frequencies. The use of ultrasound may induce nucleation.

Preferably the active particles which are crystallised or precipitated from the solution in the disperse phase consist essentially of the active substance. However, they may also comprise other material such as surfactant or included solvent. The inclusion of such residual surfactant in the active particles may make those particles more easily wetted and more easily dissolved which will be especially beneficial where the active substance is one which is of low solubility under conditions obtaining in parts of the digestive tract. It may in some cases be desired to form active particles comprising an active agent in combination with one or more other materials such as excipients.

In that case, the excipient may be added to the solution comprising the active substance in which case it may be co-crystallised with the active substance or may adsorb to the surfaces of the active particles.

Where the active particles are isolated particles which are intended for inhalation, they may comprise an additive material for the promotion of the dispersal of the active particles into an aerosol on activation of an inhaler.

Advantageously the additive material is an anti-adherent material and will tend to decrease the cohesion between the active particles and between active particles and any carrier particles. In order to determine whether a given material is an anti-adherent material, the test described in International Specification WO97/03649 (pages 6 and 7) using an "Aeroflow" apparatus may be used, anti-adherent materials being those additive materials that result in a lowering of the mean time between avalanches of the powder, as compared with the powder in the absence of the additive material.

Advantageously the additive material is an anti-friction agent (glidant) and will give better flow of powder in the dry powder inhaler which will lead to a better dose reproducibility from the inhaler device.

Where reference is made to an anti-adherent material, or to an anti-friction agent, the reference is to include those materials which will tend to decrease the cohesion between the active particles and the carrier particles, or which will tend to improve the flow of powder in the inhaler, even though they may not usually be referred to as anti-adherent material or an anti-friction agent. For example, leucine is an anti-adherent material as herein defined and is generally thought of as an anti-adherent material but lecithin is also an anti-adherent material as herein defined, even though it is not generally thought of as being anti-adherent, because it will tend to decrease the cohesion between the active particles and the carrier particles. The additive material will usually consist of physiologically acceptable material and it is also highly preferable for the additive material to be a material which may be safely inhaled into the lower lung where it may be absorbed into the blood stream.

The additive material may include a combination of one or more materials.

Advantageously, the additive material includes one or more compounds selected from amino acids and derivatives thereof, and pharmaceutically inactive peptides and polypeptides having molecular weight from 0.25 to 100Kda, and derivatives thereof. Amino acids, peptides or polypeptides and their derivatives are both physiologically acceptable and give acceptable release of the active particles on inhalation.

It is particularly advantageous for the additive material to comprise an amino acid. Amino acids have been found to give, when present in low amounts in a powder as additive material, high respirable fraction of the active materials with little segregation of the powder and also with very little of the amino acid being transported into the lower lung. The additive material may comprise one or more of any of the following amino acids: leucine, isoleucine, lysine, valine, methionine, phenylalanine. The additive may be a salt of a derivative of an amino acid, for example aspartame or acesulfame K. Preferably, the additive particles consist substantially of leucine, advantageously L-leucine. Leucine has been found to give particularly efficient release of the active particles on inhalation. The L-, D- and DL-forms of amino acids may be used.

The additive material may comprise particles of a phospholipid or a derivative thereof. Lecithin has been found to be a good material for the additive material.

The additive material may include or consist of one or more surface active materials, in particular materials that are surface active in the solid state, which may be water soluble, for example lecithin, in particular soya'lecithin, or substantially water insoluble, for example solid state fatty acids such as lauric acid, palmitic acid, stearic acid, erucic acid, behenic acid, or derivatives (such as esters and salts) thereof. Specific examples of such materials are: magnesium stearate; sodium stearyl fumarate; sodium stearyl lactylate; phospatidylcholines, phosphatidylglycerols and other example of natural and synthetic lung surfactants; liposomal formulations; lauric acid and its salts, for example, sodium lauryl sulphate, magnesium lauryl sulphate; triglycerides such as Dynsan 118 and Cutina HR; and sugar esters in general.

Magnesium stearate is a preferred additive material.

Other possible additive materials include talc, titanium dioxide, aluminium dioxide, silicon dioxide and starch.

Advantageously, the active particles will comprise at least 30%, preferably at least 50%, more preferably at least 80% and especially preferably at least 90% w/w of the active substance.

A known phenomenon displayed by emulsions is the gradual transfer of material from smaller droplets to larger droplets as the emulsion adjusts to achieve a minimum total free surface energy. The same process (Ostwald ripening) also takes place within the solid particles. Thus, after the particles have been formed, active material dissolving from the surfaces of the particles will be preferentially deposited on larger particles, leading to a gradual disappearance of smaller particles and a reduction of the polydispersity of the particle size distribution. The rate at which Ostwald ripening occurs will depend on many factors including the temperature and the composition of the emulsion. Preferably, after the formation, in the dispersed phase, of solid particles, the solid particles undergo Ostwald ripening.

The composition may be the emulsion containing the active particles which may be used directly as a medicament or, more likely, may be used as an ingredient in a medicament formulation. Alternatively, the active particles are isolated to give a dry powder composition consisting of isolated active particles and that powder is used as a medicament or as an ingredient in a medicament formulation.

Thus, the method may comprise a step in which the solid particles are isolated. The isolation step may involve the addition of a flocculant such as a polymeric flocculant. The method may involve centrifugation, filtration, distillation, air flotation or spray drying.

A preferred method of isolating the active particles involves addition to the emulsion of further amounts of either the solvent or more preferably the continuous phase to reduce the viscosity followed by filtration. Preferably the emulsion is broken prior to filtration, for example, by freeze/thaw cycling, or by ultrasound.

Another method of isolating the active particles is to dissolve the emulsion in a large volume of solvent (which should be so chosen that it will not dissolve the active particles) followed by evaporation. That evaporation stage will, of course, not take place until after the active particles have been formed. A further possible method is to isolate the active particles by spray drying the emulsion, after the active particles have been formed.

Where the continuous phase and/or the solvent are volatile, for example having a boiling point below 20°C, the active particles may be isolated by allowing the volatile liquids to evaporate off into the atmosphere. Suitable volatile liquids include n-butane, propane, isobutane, dimethylether, chlorofluorocarbon 12, hydrofluorocarbon 134a, hydrofluorocarbon 227, HCFC-22, HCFC-123 and HCFC-124 and hydrofluoroalkanes such as HFA-125 (pentafluoroethane), HFA-152 (difluoroethane).

After isolation the particles may be washed and dried, for example, to remove residual surfactant and solvent. The wash liquid should be so chosen to dissolve the residual surfactant and/or solvent but not the active particles. More than one washing stage may be required.

The invention also provides a method of making active particles comprising an active substance, the method comprising the steps of:

a) providing an emulsion comprising a solution of the active substance; and
b) inducing the formation of solid particles comprising the active substance.

The invention further provides a composition comprising active particles comprising an active substance, the composition being obtainable by any of the methods according to the invention described above.

The active substance is present in the particles as a solid material and preferably is present in the form of crystals consisting essentially of the active substance. Those crystals may be present in combination with an amorphous i.e. non-crystalline fraction of the active substance. The active substance may be present as a solid, intimate mixture with other materials such as excipients. Active particles isolated from the emulsion may also comprise some residual surfactant. If desired, for example, where the surfactant is not pharmaceutically acceptable, the particles may be washed to remove that surfactant.

It will be understood that, when the context requires, for example, when referring to particle size distributions, the term "particles" as used herein should be taken to refer to a collection of a large number of particles such as in a powder or a suspension.

It is known that fine particles prepared by micronisation (milling) or spray drying have an amorphous, that is, a non-crystalline component. The amorphous component is associated with an increase in surface free energy which is in turn associated with poor handling characteristics and, when the particles are used in powders for inhalation, the poor aerosolisation.

The particles according to the invention may have a higher degree of crystallinity, especially in the region of the surface, than particles prepared by micronisation or spray drying. The particles may therefore have a reduced surface free energy which will lead to improved handling characteristics. The surface free energy may be measured by microcalorimetry, dynamic vapour sorption or inverse gas chromatography.

The active particles may substantially consist of single crystals. The active particles may consist of aggregates of crystals. Those crystals will have grown together during the formation of the particle and the aggregate will therefore be mechanically stable.

Preferably, the active particles have a low polydispersity, especially as compared to the polydispersity of particles formed by known rapid crystallisation/ precipitation methods.

One property related to the particle size distribution of a collection of particles is the kurtosis and the approach of using Kurtosis to describe a particle size distribution is well established in the pharmaceutical sciences (see, for example, Staniforth J. N. (1988), Pharmaceutics The Science of Dosage Form Design, Ed. Aulton, M.E., Churchill Livingstone, ISBN:0443036438.

The symmetry of a distribution is based on a comparison of the height or thickness of the tails of the distribution curve and the "sharpness" of the peaks with those of a normal distribution. "Thick" tailed, "sharp" peaked curves are described as leptokurtic whereas "thin" tailed, "blunt" peaked curves are platykurtic and the normal distribution is mesokurtic.

The normalised coefficient of kurtosis, (defined in formula (2) shown below), has a value of 0 for a mesokurtic normal distribution, a negative value for curves showing platykurtosis and positive values for leptokurtic size distributions.

For univariate data, the formula for Kurtosis (K) is

$$k = \frac{n\Sigma(x\text{-}\mu)^4}{(n\text{-}1)\sigma^4}$$

$n$ = number of points
$x$ = particle size
$\mu$ = mean particle size
$\sigma$ = standard deviation

The Kurtosis of a standard mesokurtic normal distribution is three. Therefore the formula can be normalized as shown below so that a mesokurtic distribution has a value of zero. Normalised kurtosis =

$$\frac{n\Sigma(x\text{-}\mu)^4}{(n\text{-}1)\sigma^4} \text{ -3}$$

Thus positive normalised kurtosis values are indicative of a peaked (leptokurtic) distribution whereas negative normalised kurtosis values are indicative of a flat (platykurtic) distribution.

Thus negative normalised Kurtosis represents a relatively flat distribution (i.e. a wide particle size distribution) and positive normalised Kurtosis is indicative of a narrow size distribution.

The normalized Kurtosis is defined in formula (2) above and can be derived for a given particle size distribution by using any of a number of widely available statistical software packages, for example, Microsoft Excel.

A large number of data points is required to provide an accurate analysis of the Kurtosis of a particle size distribution. Indeed a true Kurtosis value would require the exact size of every particle in a distribution. However we have found that carrying out a normalised Kurtosis measurement of the values in the classes of a commercial particle sizing apparatus can give a good measure of the degree of particle size polydispersity in a sample.

Using the Malvern Mastersizer 2000 we have examined the particle size by volume of a range of materials. The Mastersizer 2000 utilises a log-normal scale for description of particle sizes. We have then examined the data statistically to determine normalized Kurtosis by copying the particle size data into Microsoft Excel and performing the "KURT" function according to the method given in Example 1. Kurtosis values for those materials are given in Table 1 along with volume median diameters. A mesokurtic normal distribution has a normalized Kurtosis value of zero.

Table 1:

| Results of particle size and Kurtosis analysis of a range of materials. | | | |
|---|---|---|---|
| Sample Name | Brief Description | d(0.5) | Normalised Kurtosis |
| Paracetamol | Sigma Paracetamol | 19 | -0.43 |
| Paracetamol | from Example 1 | 21 | 6.93 |
| Paracetamol | Milled Sigma | 12 | -1.10 |
| GattephenT250 | Fractionated paracetamol | 368 | 12.75 |
| Tastemasked Paracetamol | Tastemasked paracetamol | 210 | 4.05 |
| Gattaphen fine | Tastemasked paracetamol | 216 | 7.32 |
| Microcaps | Tastemasked paracetamol | 414 | 11.05 |
| 300nm Latex beads | Monosized Spheres | 0.304 | 61.03 |
| 0.99um Latex | Monosized Spheres | 1.014 | 60.74 |
| 8.9um Latex | Monosized Spheres | 8.802 | 50.53 |

We have found that the size distributions for pharmaceutical materials having a relatively large particle size are often positive. For example one brand of tastemasked paracetamol had a normalized Kurtosis of 3.8, another had one of 4.1. However these materials had volume median diameters of 190 and 200μm, respectively. When smaller particles of paracetamol were examined it appeared that the control of particle size distribution was difficult to achieve. A commercial grade of paracetamol (with a volume median particle size of 19μm) had a normalized Kurtosis of -0.43, indicating the presence of both very fine and relatively large particles. These would be expected to cause dissolution problems because fine particles will, in general, dissolve more quickly than larger particles of the same material due to their higher surface area to volume ratio and so the period from the dissolution of the finest particles to the final dissolution of the largest particles will be relatively long. It will therefore be appreciated that a powder having a narrow size distribution would dissolve over a time period which is relatively short and well defined compared to a powder of the same material having a broader size distribution. An attempt to reduce the number of the larger particles by milling in a mortar and pestle reduced the volume median diameter (to 12μm) but the normalized Kurtosis was then found to be -1.1. This indicates that the milling had resulted in a broader spread of particle sizes.

In contrast, paracetamol material according to the invention had a normalized Kurtosis of 6.9 with a volume median particle size of 12μm. That material should have more predictable dissolution characteristics.

Almost truly monosized particles are measured on the Mastersizer as having a normalized Kurtosis of 50 or more.

The invention also provides a composition comprising active particles comprising an active substance and having a median diameter of less than 100μm and having a normalized Kurtosis of at least 5. Preferably, the particles have a normalised Kurtosis of at least 6, more preferably 8 and especially preferably 10 and most preferably 20.

Preferably, the active particles have a mass median diameter of not more than 100μm. The active particles may have a mass median diameter of not more than 50μm, for example, in the range of 50 to 1μm which would be suitable for tabletting. Where the active particles are intended to be inhaled, they will preferably have a mass median aerodynamic diameter of less than 10μm, more preferably less than 5μm. For some applications, it will be desired for the active particles to have a diameter of about 1μm or less. Preferably, the particles will have a mass median diameter of more than 0.01μm.

Preferably the active particles have a Carr's index of less than 30, advantageously less than 20, especially advantageously less than 15 and most preferably less than 10.

The composition may be an emulsion in which the active particles are suspended. The composition may be a dry powder which consists essentially of the active particles.

The composition comprising the active particles can be used in any form of drug administration which utilizes small drug particles.

The active particles may be suitable for use in an inhaler, which may be a metered dose inhaler or a dry powder inhaler and the invention provides an inhaler containing active particles as described above. Isolated active particles may be used directly as a powder for use in a dry powder inhaler. Optionally, further materials such as excipients, taste modifiers and additive materials to promote the formulation of an aerosol on activation of an inhaler may be included in minor amounts. Suitable formulations, which contain a major proportion of the active particles and such an additive material are described in WO97/03649. Another type of dry powder formulation comprises a major proportion of carrier particles. The carrier particles are often large particles greater than 90μm in diameter to give good flow properties to the powder and consist of a pharmaceutically inert material such as a crystalline sugar, for example lactose. Those formulations may also comprise further materials such as taste modifiers and additive materials to promote the release of the active particles from the surfaces of the carrier particles on actuation of the inhaler. Dry powder formulations comprising carrier particles and such additive materials are described in WO96/23485. Alternatively, the active particles may be included in a propellant based formulation for use in a pressurized metered dose inhaler (pMDI).

In general, the skilled person will be aware of suitable formulations for inhalation and will be able to include the active particles of the invention in such formulations without difficulty.

The active particles may be suitable for use in a needleless injection device.

The active particles may be suitable for use in the manufacture of a medicament for oral ingestion such as a tablet.

The active particles may be suitable for use in a hard gelatin capsule. The active particles may be suitable for use in a soft gelatin capsule.

Tablets are made by compression of a mixture of the pharmaceutically active substance or substances with one or more other components. Those other components may include, for example, carriers, binders, lubricants and other excipients or additives. The invention provides a tablet comprising active particles according to the invention. Another common form of medicament for oral ingestion is the capsule. Capsules, which may be of the hard or soft type, have a shell which may be composed of, for example hydroxpropylmethyl-cellulose or gelatine. Capsules may be filled with granules, a powder or a liquid containing the active substance(s). The invention also provides a capsule comprising active particles according to the invention.

Delivery of a medicament by oral ingestion has the advantages that delivery is non-invasive and that the quantity of the drug administered can be relatively accurately controlled. Where the active substance is one that is of low solubility under the conditions obtaining in the digestive tract, however, full dissolution may not occur during the passage of the active substance through the digestive tract (approximately 48 hours) with the result that only a proportion of the administered amount of the active substance will become fully available for absorption into the body. That represents a waste of active substance, which is often expensive, but more importantly creates uncertainty over the precise amount of active compound absorbed by the patient. Furthermore, the slow rate of absorption into the body may undesirably delay the therapeutic effect of the active compound. It is believed that the active particles according to the invention will have more predictable dissolution characteristics.

The active particles may be suitable for use in an oral powder delivery device.

Embodiments of the invention will now be described by way of example with reference to the accompanying drawings of which:

Figure 1    shows an apparatus for preparing the composition according to the invention;
Figure 2    shows the particle size distribution of a sample of active particles according to the invention compared to a sample of commercially available particles of the same material;
Figure 3    shows particles of paracetamol obtained in Example 1; and

Figure 4     shows the particles of Figure 3 at higher magnification.

Example 1

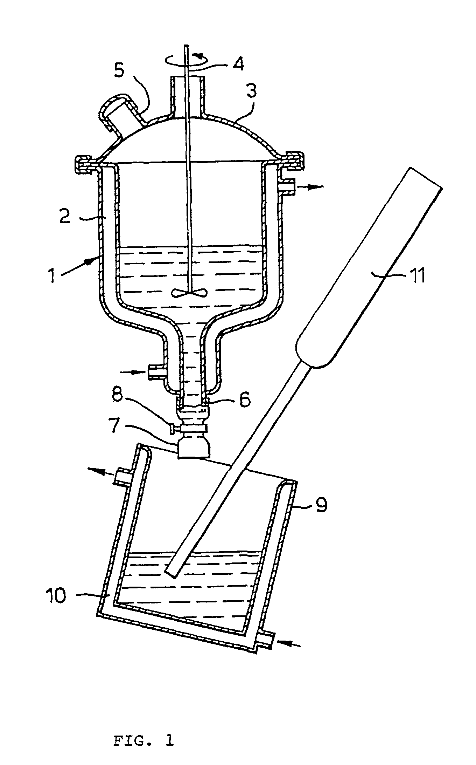

An emulsion of the following composition was prepared using the apparatus shown in Fig 1.

| Oil phase | |
|---|---|
| Soybean oil | 36.6% by weight |
| Sorbitan monooleate | 5.3% by weight |
| Poly(oxyethylene) hydrogenated castor oil | 1.1% by weight |
| | |
| Aqueous phase | |
| Deionised water (Milli-Q) | 55.3% by weight |
| Paracetamol (Sigma) | 1.7 % by weight |

The apparatus shown in Fig 1 comprises an upper vessel of volume 500cm$^3$ for holding the disperse phase. The upper vessel (1) has a temperature controlled water jacket (2), a lid (3) having a central port for stirrer shaft (4) and a port (5) for addition of material. In the bottom of the upper vessel (1) is outlet (6) over which is fitted a length of PVC tubing (7) having a clip (8) to act as a flow control.

Fixed below the outlet (6) is a lower vessel (9) for holding the continuous phase initially and the emulsion when formed. The lower vessel (9) also has a water jacket (10). Homogeniser (11) is arranged to stir the contents of the lower vessel (9).

The water was placed in the upper vessel (1) and heated to 70°C. The paracetamol was then added to the water via the small neck in the upper vessel lid and the solution was stirred until all the paracetamol dissolved. The oil phase was added to the lower vessel (9) and heated to 70°C. The homogeniser (11) was started at 16000 rpm and the clip (8) on the PVC tubing (7) was loosened to allow a slow drip of the aqueous phase into the oil. The clip was gradually loosened over time so that the flow was increased as the emulsion began to form. Once all the aqueous phase had been added the speed of homogenisation was increased to maximum of 24000 rpm for a few minutes. The emulsion was then cooled at a rate of 20°C per hour, with low speed homogenisation. The particles were isolated by filtering the emulsion under vacuum and were washed with a little cold water. The particle size distribution of the particles was measured with a Malvern Mastersizer by the following method:

(i) The active particles were suspended (using stirring and sonication) in cyclohexane with 0.1% lecithin. Sufficient active particles were used to ensure the necessary obscuration;
(ii) The suspension was placed in the wet cell of the Malvern Mastersizer 2000 and the particle size distribution was measured using an appropriate lens system;
(iii) The results were analysed using the proprietary software of the Mastersizer 2000 to produce the size distribution given in Table 2.
(iv) The results shown in Table 2 were analysed using the KURT function of Microsoft Excel which gave the normalized Kurtosis value of 6.93.

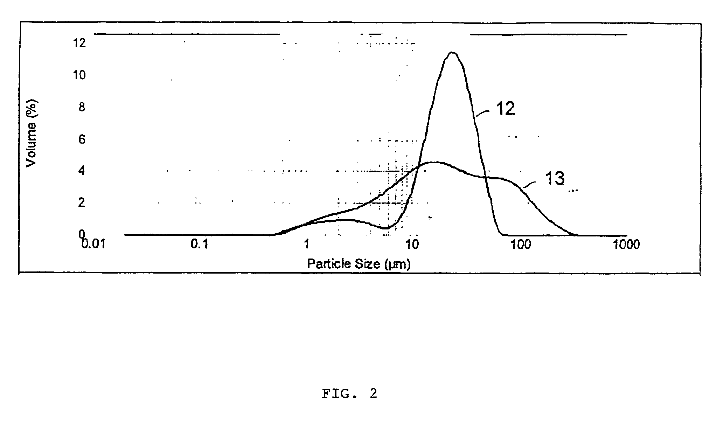

The size distribution is shown in Fig 2 as line 12 along with the particle size distribution of a sample of the commercially available (Sigma) paracetamol used as the starting material (line 13). It can be seen that the particles according to the invention have a much reduced polydispersity as compared to the starting material.

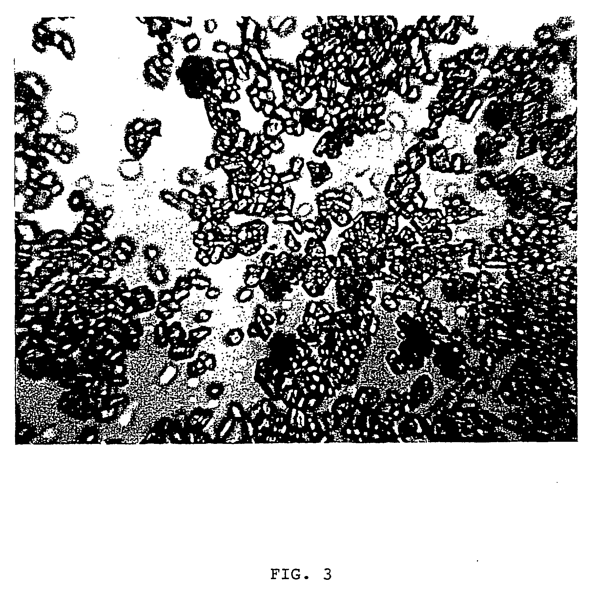

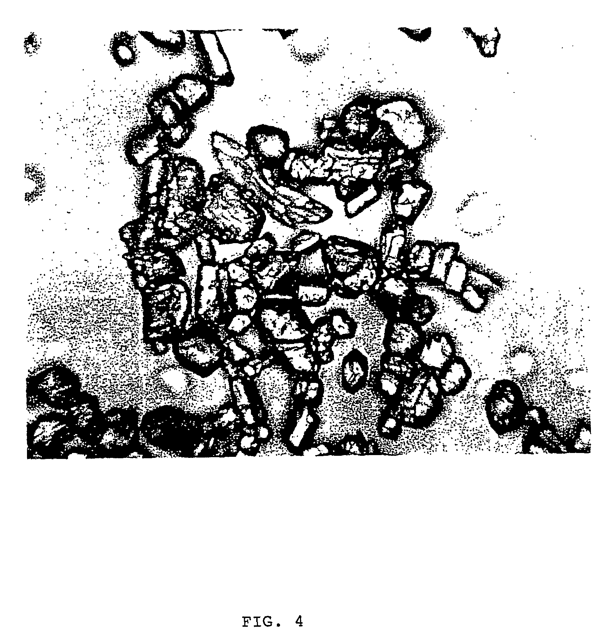

Photomicrographs of the isolated paracetamol particles made according to the above method are shown in Figures 3 and 4.

| Size (µm) | Volume in % |
|---|---|
| 0.010 | 0.00 |
| 0.011 | 0.00 |
| 0.013 | 0.00 |
| 0.015 | 0.00 |
| 0.017 | 0.00 |
| 0.020 | 0.00 |
| 0.023 | 0.00 |
| 0.026 | 0.00 |
| 0.030 | 0.00 |
| 0.035 | 0.00 |
| 0.040 | 0.00 |
| 0.046 | 0.00 |
| 0.052 | 0.00 |
| 0.060 | 0.00 |
| 0.069 | 0.00 |
| 0.079 | 0.00 |
| 0.091 | 0.00 |
| 0.105 | 0.00 |
| 0.120 | 0.00 |
| 0.138 | 0.00 |
| 0.158 | 0.00 |
| 0.182 | 0.00 |
| 0.209 | 0.00 |
| 0.240 | 0.00 |
| 0.275 | 0.00 |
| 0.316 | 0.00 |
| 0.363 | 0.00 |
| 0.417 | 0.00 |
| 0.479 | 0.00 |
| 0.550 | 0.14 |
| 0.631 | 0.26 |
| 0.724 | 0.39 |
| 0.832 | 0.48 |
| 0.955 | 0.57 |
| 1.096 | 0.64 |
| 1.259 | 0.70 |
| 1.445 | 0.75 |
| 1.660 | 0.78 |
| 1.905 | 0.81 |
| 2.188 | 0.82 |
| 2.512 | 0.81 |
| 2.884 | 0.78 |
| 3.311 | 0.70 |
| 3.802 | 0.60 |
| 4.365 | 0.48 |
| 5.012 | 0.39 |
| 5.754 | 0.41 |
| 6.607 | 0.61 |
| 7.586 | 1.11 |
| 8.710 | 1.95 |
| 10.000 | 3.17 |
| 11.482 | 4.67 |
| 13.183 | 6.36 |
| 15.136 | 7.98 |
| 17.378 | 9.34 |
| 19.953 | 10.15 |
| 22.909 | 10.28 |
| 26.303 | 9.66 |
| 30.200 | 8.38 |
| 34.674 | 6.65 |
| 39.811 | 4.72 |
| 45.709 | 2.88 |
| 52.481 | 1.39 |
| 60.256 | 0.18 |
| 69.183 | 0.01 |
| 79.433 | 0.00 |
| 91.201 | 0.00 |
| 104.713 | 0.00 |
| 120.226 | 0.00 |
| 138.038 | 0.00 |
| 158.489 | 0.00 |
| 181.970 | 0.00 |
| 209.930 | 0.00 |
| 239.883 | 0.00 |
| 275.423 | 0.00 |
| 316.228 | 0.00 |
| 363.078 | 0.00 |
| 416.869 | 0.00 |
| 478.630 | 0.00 |
| 549.541 | 0.00 |
| 630.957 | 0.00 |
| 724.436 | 0.00 |
| 831.764 | 0.00 |
| 954.993 | 0.00 |
| 1096.478 | 0.00 |
| 1259.925 | 0.00 |
| 1258.925 | 0.00 |
| 1445.440 | 0.00 |
| 1659.587 | 0.00 |
| 1905.461 | 0.00 |
| 2187.762 | 0.00 |
| 2511.886 | 0.00 |
| 2884.032 | 0.00 |
| 3311.311 | 0.00 |
| 3801.894 | 0.00 |
| 4365.158 | 0.00 |
| 5011.872 | 0.00 |
| 5754.399 | 0.00 |
| 6606.934 | 0.00 |
| 7585.776 | 0.00 |
| 8709.635 | 0.00 |
| 10000.000 | 0.00 |

Table 2: Malvern output suitable for Kurtosis analysis in Microsoft Excel

Example 2

Particles of a biological macromolecule, bovine serum albumin (Molecular weight = 67,000) are prepared by the following method.

The following materials are used:

1) An aqueous buffered solution of bovine serum albumin (BSA), prepared by dissolving 60mg/cm$^3$ of the BSA in a 50mM sodium acetate (NaAC) buffer at a pH of 5.0.
2) A saturated solution of the precipitating substance - ammonium sulphate, prepared in an aqueous 50mM sodium acetate buffer at a pH of 5.0.
3) An oily Isopropyl myristate.
4) A surfactant, dioctyl sulphosuccinate sodium salt.

Deionized water is used in the preparation of all aqueous solutions.

Two separate microemulsions are prepared at ambient temperature in 30cm$^3$ vials. For each microemulsion, 5g of the surfactant and 10g of the oil are combined and rapidly stirred. In preparing each water-in-oil microemulsion, equal volumes of the aqueous solution of BSA and the ammonium sulphate solution are added dropwise to the rapidly stirred surfactant-oil mixtures.

The amount of aqueous solutions mixed in each microemulsion are chosen to obtain the desired molar ratio of the water to surfactants, R = [water/surfactant] for example R = 25. The radius of the dispersive water pool in the continuous oil phase may be changed by varying R. R is preferably in the range of 20 to 56.

After formation of two microemulsions, the two water-in-oil microemulsions are rapidly mixed together in a 100ml vial. Due to the exchange process which subsequently occurs between droplets (described below), the mixing of the microemulsions results in size-controlled crystallites of the bovine serum albumin by precipitation of the protein within the water droplets.

It is possible to control the crystal form, and shape and size of the protein particles by varying the concentration of the solution of ammonium sulphate. Also, for proteins with a temperature-dependent solubility, the temperature of the vials can be controlled to combine precipitation with crystallisation of the protein. For example, if the temperature of the mixed microemulsion is maintained at between 8 and 17°C spherical agglomerates are formed, whereas if the temperature is maintained between 18 and 37°C, non-spherical crystals are formed. The particles are isolated by filtration. The concentration of surfactant is then reduced by washing the particles in an excess of the ammonium sulphate solution.

Dynamics of exchange process

Since the precipitation in a mixed water-in-oil microemulsion is confined within the dispersed water droplets, a necessary step prior to precipitation is the transfer of the reactants into the same droplet. The readiness with which that process occurs in any given system is determined by the inter-micellar exchange rate constant, $k_{ex}$ and the diffusion-controlled droplet collision, $k_{diff}$. These rates of exchange and diffusion are a function of each particular water-in-oil emulsion system, and can be controlled by varying the temperature, nature and/or amount of surfactants and by adding additive substances.

Example 3

This example illustrates the use of an emulsion crystallisation technique, which utilises the temperature-solubility dependence of paracetamol to crystallise paracetamol particles/crystals having a low polydispersity.

The following liquid phases are used:

1. A saturated solution of paracetamol, prepared by dissolving 65g of paracetamol in 100g of deionized water at 70°C. When saturated, the solution is filtered into the reservoir vessel. To avoid any unwanted precipitation/crystallisation the solution is maintained at ~70°C which is a few degrees above the saturation point of the solution.
2. An oil phase containing soybean oil and surfactants - polyoxyethylene castor oil derivative and sorbitan monooleate. 41g of soybean oil, 1.5g of polyoxyethylene caster oil derivative and 7.5g of sorbitan monooleate are combined in a temperature controlled jacketed vessel, and forcefully stirred at 70°C.

150g of the saturated paracetamol solution is added dropwise, via a capillary tube, at a controlled flow rate into the stirred oil. Upon formation of the emulsion, under intense stirring, paracetamol crystallisation is induced within the water droplets by lowering the temperature of the emulsion. Control of crystallisation can be achieved both by varying the temperature drop between the saturated and crystallisation temperatures, and by using a predetermined temperature ramp in the crystallisation vessel thereby controlling the nucleation and subsequent growth of the paracetamol particles/crystals.

The amount of paracetamol solution mixed in the emulsion is chosen to obtain the desired molar ratio of water to surfactants, R=[water]/[surfactant], for example, R = 25. The radius of the dispersive water pool that is, the radius of the droplets, may be changed by varying R. Preferably, R is in the range of from 25 to 56.

The crystals of paracetamol are separated from the emulsion by filtration under vacuum and washing with a suitable solvent. Alternatively, the volatile components of the emulsion may be removed by distillation.

Example 4

This example illustrates the use of an oil in water emulsion of the following composition:

| Castor oil (saturated with Beclomethasone dipropionate) | 15% by weight |
| Sorbitan monooleate | 4.25% by weight |
| Polyoxyethylene-(20)-sorbitan monooleate | 0.75% by weight |
| Water | 80% by weight |

The surfactants (sorbitan monooleate and polyoxyethylene-(20)-sorbitan monooleate) are dissolved in the castor oil/Beclomethasone dipropionate at 70°C. The water is heated to 70°C and the castor oil/surfactant mixture added with stirring at about 700 rpm. The emulsion is homogenised for 1 minute using a high shear mixture and then cooled to room temperature under continued stirring.

The formation of solid particles of the Beclomethasone dipropionate may be induced either by adding a water soluble precipitant substance, by reducing the temperature of by changing the pH.

Example 5

This example illustrates the use of a surfactant-free emulsion of the following composition:

| Soybean oil (saturated with fluticasone proprionate) | 20% by weight |
| Water | 75% by weight |
| Poly(acrylic acid) | 5% by weight |

The soybean oil and the water are separately heated to 75°C and combined under stirring at 700 rpm. The emulsion is homogenised with a high shear mixer for 1 minute and then the poly(acrylic acid) is dispersed in the emulsion with stirring.

Example 6

This example illustrates the use of a multiple emulsion, specifically a water in oil in water emulsion.
In a first stage a primary emulsion of the following composition is prepared.

| Primary Emulsion | | |
|---|---|---|
| **Water in Oil** | | |
| A | Glyceryl monostearate | 3% |
| | Sorbitan monooleate | 3% |
| | Soybean oil | 29% |
| | | |
| B | Water (saturated with terbutaline or ipratropium) | 61% |
| | NaCl | 4% |

The soybean oil and the surfactants are mixed together to form mixture A which is heated to 75°C. The sodium chloride is dissolved in the water to give solution B which is also heated to 75°C. Solution B is added to mixture A whilst stirring at 700 rpm. The resulting primary emulsion is homogenised on a high shear mixer for 1 minute and is maintained at 75°C whilst being stirred at 500 rpm.

A multiple emulsion of the following composition is then prepared.

| Secondary Emulsion | | |
|---|---|---|
| **Water (1) in Oil in Water (2)** | | |
| C | Primary emulsion | 60% |
| D | Poloxamer (POE/POP block compolymer) | 2% |
| | Water | 15% |
| E | NaCl | 2% |
| F | Water | 20% |
| | Poly(acrylic acid) | 0.2% |

The poloxamer is dissolved in water at 5°C to make solution D which is maintained at 5°C. The sodium chloride (E) and the primary emulsion are then added to solution D whilst stirring at 700 rpm to form an emulsion. As the primary emulsion cools on contact with solution D, crystallization of the terbutaline or ipratropium occurs. Solution F is then prepared by adding the poly(acrylic acid) to water until a homogenous gel is formed. F is then added in small portions to the emulsion whilst stirring at 400 rpm. Stirring at 300 rpm is continued until F is completely dispersed.

During the formation of the solid particles the oil may act as a semi permeable membrane and controls the rate of diffusion between the water droplets and the continuous phase.

It is also possible to use an oil-soluble active substance which will dissolve in the soybean oil. In that case, crystallisation may be induced from the inside of the oil droplet or from outside.

Example 7

A dry powder for inhalation is prepared as follows.

Lactose (Meggle EP D30) was sieved with sieves having 90µm and 125µm meshes to isolate particles having a diameter in the range of 90 to 125µm.

4g of leucine (having 95% of weight of particles having a diameter less than 150µm) are added to 196g of the sieved lactose carrier particles and blended together in a Turbula mixer for 15 minutes. 29.86g of the lactose and leucine mixture are mixed with 0.132g of active particles according to the invention, for example, beclomethasone diproprionate particles in a glass mortar. Capsules are then each filled with 25mg of the powder and are then fired from a Rotahaler dry powder inhaler (Glaxo). The respirable fraction of the dose of active particles may be measured using a twin stage impinger as described in WO96/23485.

**Claims**

1. A method of making a composition comprising active particles comprising an active substance, the method comprising the steps of:

    a) providing an emulsion having a dispersed phase comprising a solution of the active substance in a solvent; and
    b) inducing the formation, in the emulsion, of solid particles comprising the active substance, thereby forming an emulsion comprising the solid active particles.

2. A method as claimed in claim 1, wherein the dispersed phase is in the form of droplets having a mean droplet diameter of at least 1µm.

3. A method as claimed in claim 1, which includes the steps of:

    combining the active substance with the solvent to form the solution and then
    combining the solution with the continuous phase to form the emulsion.

4. A method as claimed in any of claims 1 to 3, in which the emulsion comprises one or more surfactants, and/or the solvent is substantially insoluble in the continuous phase.

5. A method as claimed in any one of claims 1 to 4, in which the dispersed or the continuous phase is an aqueous phase.

6. A method as claimed in any of claims 1 to 5, in which the step of inducing the formation, in the emulsion, of solid particles involves changing the temperature of the emulsion.

7. A method as claimed in any of claims 1 to 6, in which the step of inducing the formation, in the emulsion, of solid particles involves the addition of a precipitant substance.

8. A method as claimed in claim 7, in which the precipitant substance is a substance which will combine with the active substance to form an insoluble product comprising the active substance, and/or is a liquid which is miscible with the solvent but which is a non-solvent for the active substance.

9. A method as claimed in claim 7, in which the addition of the precipitant substance causes a change in the pH of the emulsion.

10. A method as claimed in any of claims 7 to 9, in which the step of inducing the formation, in the emulsion, of solid particles involves the addition of a second emulsion, the dispersed phase of the second emulsion comprising the precipitant material.

11. A method as claimed in any of claims 1 to 10, in which after the formation, in the emulsion, of solid particles, the solid particles undergo Ostwald ripening.

12. A method as claimed in any of claims 1 to 11 in which the composition is an emulsion comprising the active particles.

13. A method as claimed in any of claims 1 to 11, which comprises a step in which the solid particles are isolated to provide a dry powder composition.

14. A method as claimed in claim 13 which involves the addition of a flocculant, and/or filtration.

15. A method as claimed in any of claims 13 to 14, in which the solid particles are washed.

16. A method as claimed in any of claims 1 to 15 which is substantially a continuous process.

17. A composition comprising active particles comprising an active substance, the composition being obtainable by the method of any of claims 1 to 16.

18. A composition comprising active particles comprising an active'substance and having a median diameter of less than 100µm, a normalised Kurtosis of at least 5 or 6 and, optionally, a median diameter of not more than 50µm.

19. A composition as claimed claim 17 or 18 comprising active particles which consist essentially of the active substance.

20. A composition as claimed in any of claims 17 to 19 comprising active particles which have a high degree of crystallinity and, optionally, a higher degree of crystallinity in the region of the surface.

21. A composition as claimed in any of claims 17 to 20 comprising active particles which consist substantially of single crystals, or which consist of aggregates of crystals.

22. A composition comprising active particles as claimed in any of claims 17 to 21 having a mean diameter of less than 1µm.

23. A composition as claimed in any of claims 17 to 22 which is an emulsion, or which consists of isolated active particles.

24. A composition as claimed in claim 18 obtainable by the method as claimed in any of claims 1 to 16.

25. A composition as claimed in claim 23 which consists of isolated active particles and is suitable for use in an inhaler, a transdermal injection device, the manufacture of a tablet, an oral powder delivery device, and/or a gelatin capsule.

**26.** A dry powder inhaler or a tablet comprising a composition as claimed in claim 23.

**Patentansprüche**

**1.** Verfahren zur Herstellung einer Zusammensetzung umfassend wirksame Partikel, die eine wirksame Substanz umfassen, wobei das Verfahren die Schritte umfaßt des:

a) Bereitstellens einer Emulsion, die eine dispergierte Phase aufweist, die eine Lösung der wirksamen Substanz in einem Lösungsmittel umfaßt; und
b) Auslösens der Bildung von festen, die wirksame Substanz umfassenden, Partikeln in der Emulsion, wobei eine Emulsion gebildet wird, die die festen wirksamen Partikel umfaßt.

**2.** Verfahren nach Anspruch 1, wobei die dispergierte Phase in der Form von Tröpfchen mit einem mittleren Tröpfchendurchmesser von mindestens 1 μm vorliegt.

**3.** Verfahren nach Anspruch 1, das die Schritte beinhaltet des:

Zusammenbringens der wirksamen Substanz mit dem Lösungsmittel zur Bildung der Lösung, und des darauf folgenden Zusammenbringens der Lösung mit der kontinuierlichen Phase zur Bildung der Emulsion.

**4.** Verfahren nach einem der Ansprüche 1 bis 3, bei dem die Emulsion ein oder mehrere Tenside umfaßt, und/oder das Lösungsmittel in der kontinuierlichen Phase im wesentlichen unlöslich ist.

**5.** Verfahren nach einem der Ansprüche 1 bis 4, bei dem die dispergierte oder die kontinuierliche Phase eine wäßrige Phase ist.

**6.** Verfahren nach einem der Ansprüche 1 bis 5, bei dem der Schritt des Auslösens der Bildung von festen Partikeln in der Emulsion eine Änderung der Temperatur der Emulsion beinhaltet.

**7.** Verfahren nach einem der Ansprüche 1 bis 6, bei dem der Schritt des Auslösens der Bildung von festen Partikeln in der Emulsion das Zufügen eines Fällungsmittels beinhaltet.

**8.** Verfahren nach Anspruch 7, bei dem das Fällungsmittel eine Substanz ist, die sich mit der wirksamen Substanz zur Bildung eines unlöslichen Produktes, umfassend die wirksame Substanz, verbinden wird, und/oder eine Flüssigkeit ist, die mit dem Lösungsmittel mischbar ist, die jedoch kein Lösungsmittel für die wirksame Substanz ist.

**9.** Verfahren nach Anspruch 7, bei dem das Zufügen des Fällungsmittels eine Änderung des pH der Emulsion hervorruft.

**10.** Verfahren nach einem der Ansprüche 7 bis 9, bei dem der Schritt des Auslösens der Bildung von festen Partikeln in der Emulsion das Zufügen einer zweiten Emulsion beinhaltet, wobei die dispergierte Phase das Fällungsmittel umfaßt.

**11.** Verfahren nach einem der Ansprüche 1 bis 10, bei dem, nach der Bildung von festen Partikeln in der Emulsion, eine Ostwald-Reifung der festen Partikel erfolgt.

**12.** Verfahren nach einem der Ansprüche 1 bis 11, bei dem die Zusammensetzung eine Emulsion ist, die die wirksamen Partikel umfaßt.

**13.** Verfahren nach einem der Ansprüche 1 bis 11, das einen Schritt umfaßt, bei dem die festen Partikel isoliert werden, um eine Trockenpulverzusammensetzung bereitzustellen.

**14.** Verfahren nach Anspruch 13, das das Zufügen eines Flockungsmittels und/oder eine Filtration beinhaltet.

**15.** Verfahren nach einem der Ansprüche 13 bis 14, bei dem die festen Partikel gewaschen werden.

**16.** Verfahren nach einem der Ansprüche 1 bis 15, das ein im wesentlichen kontinuierlicher Prozess ist.

**17.** Zusammensetzung, umfassend wirksame Partikel, die eine wirksame Substanz umfassen, wobei die Zusammensetzung durch ein Verfahren gemäß einem der Ansprüche 1 bis 16 erhältlich ist.

**18.** Zusammensetzung, umfassend wirksame Partikel, die eine wirksame Substanz umfassen und einen medianen Durchmesser von weniger als 100 μm, eine normalisierte Kurtosis von mindestens 5 oder 6 und, wahlweise, einen medianen Durchmesser von nicht mehr als 50 μm aufweisen.

**19.** Zusammensetzung nach Anspruch 17 oder 18, umfassend wirksame Partikel, die im wesentlichen aus der wirksamen Substanz bestehen.

**20.** Zusammensetzung nach einem der Ansprüche 17 bis 19, umfassend wirksame Partikel, die einen hohen Kristallinitätsgrad und, optional, einen höheren Kristallinitätsgrad im Bereich der Oberfläche aufweisen.

**21.** Zusammensetzung nach einem der Ansprüche 17 bis 20, umfassend wirksame Partikel, die im wesentlichen aus einzelnen Kristallen bestehen, oder die aus Aggregaten von Kristallen bestehen.

**22.** Zusammensetzung, umfassend wirksame Partikel nach einem der Ansprüche 17 bis 21 mit einem mittleren Durchmesser von weniger als 1 μm.

**23.** Zusammensetzung nach einem der Ansprüche 17 bis 22, die eine Emulsion ist oder die aus isolierten wirksamen Partikeln besteht.

**24.** Zusammensetzung nach Anspruch 18, erhältlich durch das Verfahren nach einem der Ansprüche 1 bis 16.

**25.** Zusammensetzung nach Anspruch 23, die aus isolierten wirksamen Partikeln besteht und zur Verwendung in einem Inhalator, in einer Vorrichtung zur transdermalen Injektion, zur Herstellung einer Tablette, in einer Vorrichtung zur Zuführung eines oralen Pulvers und/oder in einer Gelatinekapsel geeignet ist.

**26.** Trockenpulverinhalator oder Tablette, umfassend die Zusammensetzung nach Anspruch 23.


**Revendications**

**1.** Procédé pour la préparation d'une composition comprenant des particules actives comprenant une substance active, procédé comprenant les étapes consistant à :

a) fournir une émulsion ayant une phase dispersée comprenant une solution de la substance active dans un solvant ;
b) induire la formation, dans l'émulsion, de particules solides comprenant la substance active, en formant ainsi une émulsion comprenant les particules actives solides.

**2.** Procédé suivant la revendication 1, dans lequel la phase dispersée est sous forme de gouttelettes ayant un diamètre moyen de gouttelette d'au moins 1 μm.

**3.** Procédé suivant la revendication 1, qui comprend les étapes consistant à :

associer la substance active au solvant pour former la solution, puis
associer la solution à la phase continue pour former l'émulsion.

**4.** Procédé suivant l'une quelconque des revendications 1 à 3, dans lequel l'émulsion comprend un ou plusieurs agents tensio-actifs et/ou le solvant est pratiquement insoluble dans la phase continue.

**5.** Procédé suivant l'une quelconque des revendications 1 à 4, dans lequel la phase dispersée ou continue est une phase aqueuse.

**6.** Procédé suivant l'une quelconque des revendications 1 à 5, dans lequel l'étape consistant à induire la formation, dans l'émulsion, de particules solides comprend une variation de la température de l'émulsion.

**7.** Procédé suivant l'une quelconque des revendications 1 à 6, dans lequel l'étape consistant à induire la formation, dans l'émulsion, de particules solides comprend l'addition d'une substance précipitante.

**8.** Procédé suivant la revendication 7, dans lequel la substance précipitante est une substance qui se combinera avec la substance active pour former un produit insoluble comprenant la substance active, et/ou est un liquide qui est miscible au solvant mais qui est un non solvant pour la substance active.

**9.** Procédé suivant la revendication 7, dans lequel l'addition de la substance précipitante provoque une variation du pH de l'émulsion.

**10.** Procédé suivant l'une quelconque des revendications 7 à 9, dans lequel l'étape consistant à induire la formation, dans l'émulsion, de particules solides comprend l'addition d'une seconde émulsion, la phase dispersée de la seconde émulsion comprenant la matière précipitante.

**11.** Procédé suivant l'une quelconque des revendications 1 à 10, dans lequel, après la formation, dans l'émulsion, de particules solides, les particules solides subissent une maturation d'Ostwald.

**12.** Procédé suivant l'une quelconque des revendications 1 à 11, dans lequel la composition est une émulsion comprenant les particules actives.

**13.** Procédé suivant l'une quelconque des revendications 1 à 11, qui comprend une étape dans laquelle les particules solides sont isolées pour former une composition en poudre sèche.

**14.** Procédé suivant la revendication 13, qui comprend l'addition d'un agent floculant, et/ou une filtration.

**15.** Procédé suivant l'une quelconque des revendications 13 et 14, dans lequel les particules solides sont lavées.

**16.** Procédé suivant l'une quelconque des revendications 1 à 15, qui est essentiellement un procédé continu.

**17.** Composition comprenant des particules actives comprenant une substance active, la composition pouvant être obtenue par le procédé suivant l'une quelconque des revendications 1 à 16.

**18.** Composition comprenant des particules actives comprenant une substance active ayant un diamètre moyen inférieur à 100 $\mu$m, une valeur de Kurtosis normalisée d'au moins 5 ou 6 et, facultativement, un diamètre moyen non supérieur à 50 $\mu$m.

**19.** Composition suivant la revendication 17 ou 18, comprenant des particules actives qui consistent essentiellement en la substance active.

**20.** Composition suivant l'une quelconque des revendications 17 à 19, comprenant des particules actives qui ont un haut degré de cristallinité et, facultativement, un plus haut degré de cristallinité dans la région de la surface.

**21.** Composition suivant l'une quelconque des revendications 17 à 20, comprenant des particules actives qui consistent essentiellement en cristaux isolés, ou qui consistent en agrégats de cristaux.

**22.** Composition comprenant des particules actives suivant l'une quelconque des revendications 17 à 21, ayant un diamètre moyen inférieur à 1 $\mu$m.

**23.** Composition suivant l'une quelconque des revendications 17 à 22, qui est une émulsion, ou qui consiste en particules actives isolées.

**24.** Composition suivant la revendication 18, pouvant être obtenue par le procédé suivant l'une quelconque des revendications 1 à 16.

**25.** Composition suivant la revendication 23, qui consiste en particules actives isolées et qui est apte à l'utilisation dans un inhalateur, un dispositif d'injection transdermique, la production d'un comprimé, un dispositif d'administration orale de poudre, et/ou une capsule de gélatine.

**26.** Inhalateur de poudre sèche ou comprimé comprenant une composition suivant la revendication 23.

FIG. 1

FIG. 2

FIG. 3

FIG. 4